(19) Europäisches Patentamt
European Patent Office
Office européen des brevets

(11) Veröffentlichungsnummer : **0 253 235 B1**

(12) # EUROPÄISCHE PATENTSCHRIFT

(45) Veröffentlichungstag der Patentschrift : **02.01.91 Patentblatt 91/01**

(21) Anmeldenummer : **87109593.1**

(22) Anmeldetag : **03.07.87**

(51) Int. Cl.$^5$ : **C07D 498/06,** A61K 31/535, A23K 1/00, // (C07D498/06, 265:00, 221:00)

(54) **1,8-verbrückte 4-Chinoloncarbonsäuren und diese enthaltende Arzneimittel.**

Die Akte enthält technische Angaben, die nach dem Eingang der Anmeldung eingereicht wurden und die nicht in dieser Patentschrift enthalten sind.

(30) Priorität : **15.07.86 DE 3623757**

(43) Veröffentlichungstag der Anmeldung : **20.01.88 Patentblatt 88/03**

(45) Bekanntmachung des Hinweises auf die Patenterteilung : **02.01.91 Patentblatt 91/01**

(84) Benannte Vertragsstaaten : **AT BE CH DE ES FR GB IT LI NL SE**

(56) Entgegenhaltungen :
**EP-A- 0 047 005**
**EP-A- 0 206 076**
**PATENT ABSTRACTS OF JAPAN, Band 9, Nr. 160 (C-289)[1883], 4. Juli 1985; & JP - A - 60 34968**
**PATENT ABSTRACTS OF JAPAN, Band 7, Nr. 151 (C-174)[1296], 2. Juli 1983; & JP - A- 58 62113**
**PATENT ABSTRACTS OF JAPAN, Band 7, Nr. 167 (C-177)[1312], 22. Juli 1983; & JP - A - 58 72589**

(56) Entgegenhaltungen :
**PATENT ABSTRACTS OF JAPAN, Band 7, Nr. 53 (C-154)[1198], 3. März 1983; & JP - A - 57 - 203 085**
**PATENT ABSTRACTS OF JAPAN, Band 10, Nr. 31 (C-327)[2088], 6. Februar 1986; & JP - A - 60 - 184 014**
**PATENT ABSTRACTS OF JAPAN, Band 10, Nr. 63 (C-332)[2120], 13. März 1986; & JP - A - 60 202 822**
**Die Chemotherapeutika der Naldixinsäure-Gruppe**

(73) Patentinhaber : **BAYER AG**
**D-5090 Leverkusen 1 Bayerwerk (DE)**

(72) Erfinder : **Schriewer, Michael, Dr.**
**Am Thelen Siefen 1a**
**D-5068 Odenthal (DE)**
Erfinder : **Grohe, Klaus, Dr.**
**Am Wasserturm 10**
**D-5068 Odenthal (DE)**
Erfinder : **Hagemann, Hermann, Dr.**
**Kandinskystrasse 52**
**D-5090 Leverkusen 1 (DE)**
Erfinder : **Zeiler, Hans-Joachim, Dr.**
**Am Rohm 86**
**D-5600 Wuppertal 1 (DE)**
Erfinder : **Metzger, Karl Georg, Dr.**
**Pahlkestrasse 75**
**D-5690 Wuppertal 1 (DE)**

EP 0 253 235 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die Erfindung betrifft neue 1,8-verbrückte 4-Chinolon-3-carbonsäuren, Verfahren zu ihrer Herstellung sowie ihre Verwendung als Arzneimittel, insbesondere als antibakterielle Mittel in der Human- und Veterinärmedizin.

Gyrase hemmende, tricyclische Verbindungen auf Basis von Chinoloncarbonsäure sind unter anderem bereits aus EP-A-0 047 005, JP-A-57-203085 sowie aus "Die Chemotherapeutika der Nalidixinsäure-Gruppe", M.Caesar und W.Stille (1984) bekannt geworden. Ihre Wirksamkeit ist jedoch nicht gegenüber allen Keimen zufriedenstellend.

Die Erfindung stellt 1,8-verbrückte 4-Chinolon-3-carbonsäuren und Derivate der Formel I zur Verfügung

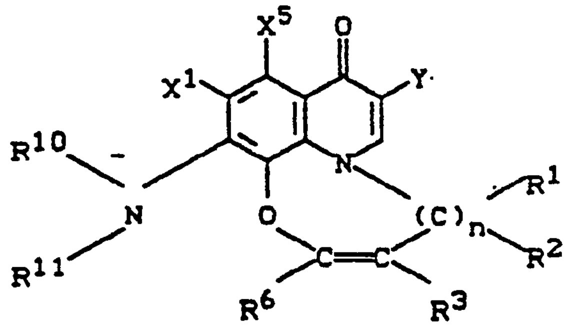

(I)

in welcher

Y für eine Carboxylgruppe steht,

$X^1$ für Fluor steht,

$X^5$ für Wasserstoff steht,

$R^{10}$ und $R^{11}$ gemeinsam mit dem Stickstoffatom, an das sie gebunden sind, einen 5- oder 6-gliedrigen heterocyclischen Ring bilden können, der als Ringglied zusätzlich ein Sauerstoffatom oder die Gruppe N-$R^{12}$ enthalten kann und der gegebenenfalls an den Kohlenstoffatomen einbis zweifach durch $C_1$-$C_2$-Alkyl, Cyclohexyl, gegebenenfalls durch Chlor, Fluor, Methyl, Phenyl, Hydroxy, Methoxy, Benzyloxy, Nitro oder Piperidino substituiertes Phenyl, 2-Thienyl oder Hydroxy substituiert sein kann, wobei $R^{12}$ für Wasserstoff, eine verzweigte oder unverzweigte Alkylgruppe mit 1 bis 3 Kohlenstoffatomen, die gegebenenfalls durch eine oder zwei Hydroxygruppen substituiert sein kann, einen Phenacylrest, einen Oxalkylrest mit bis zu 4 Kohlenstoffatomen sowie einen Rest $COR^{13}$ steht, wobei $R^{13}$ Wasserstoff oder Alkyl mit ein oder zwei Kohlenstoffatomen bedeutet,

$R_1$, $R_2$, $R_3$ und $R_6$ für Wasserstoff, eine Alkylgruppe mit vorzugsweise 1-6 Kohlenstoffatomen steht, sowie $R_6$ auch für Phenyl stehen kann und

n 0 oder 1 bedeutet

sowie die Verbindung 6,7-Difluor-9-oxo-9H-cyclohexa[1,2-b]-pyrido-[1′,2′,3′-de]1,4-benzoxacin-10-carbonsäureethylester

und deren pharmazeutisch verwendbare Hydrate oder Salze, vorzugsweise Alkali-, Erdalkali-, Silber- und Guanidiniumsalze, sowie ihre Ester.

Die Verbindungen weisen eine hohe antibakterielle Wirksamkeit auf. Sie eignen sich daher als Wirkstoffe für die Human- und Veterinärmedizin. Sie können auch als Zwischenprodukte zur Herstellung weiterer Bakterizide verwendet werden.

Die erfindungsgemäßen Verbindungen der Formel (I) erhält man, wenn man Chinoloncarbonsäurederivate der Formel (II)

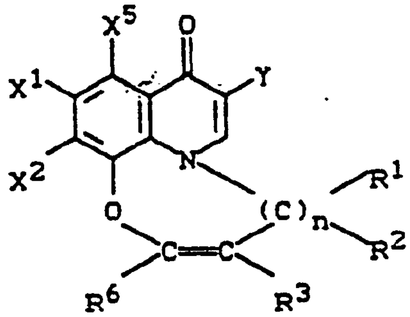

(II)

in der die Reste $X^1$, $X^5$, $R^1$, $R^2$, $R^3$ und $R^6$, Y sowie n die oben angegebenen Bedeutungen haben, sowie $X^2$ für Halogen, bevorzugt für Chlor und Fluor, steht, mit Aminen der Formel (III)

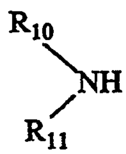

(III)

in welcher
$R^{10}$ und $R^{11}$ die oben angegebenen Bedeutungen haben, gegebenenfalls in Gegenwart von Säurebindern umsetzt (Methode A).

Dieses Verfahren muß nicht notwendigerweise so gestaltet werden, daß $R^{10}$ und $R^{11}$ in den Aminen der Formel (III) bereits ihre endgültige Bedeutung haben, die sie in den erfindungsgemäßen Verbindungen der Formel (I) haben. Es können vielmehr auch in einem ersten Schritt Vorstufen zu den Resten $R^{10}$ und $R^{11}$ benutzt werden, die dann in einem oder mehreren Folgereaktionsschritten in die endgültige Form von $R^{10}$ und $R^{11}$ überführt werden.

So können beispielsweise erfindungsgemäße Verbindungen der Formel (I) erhalten werden, indem man eine 10-(1-Piperazinyl)verbindung (bei n=0) bzw. 11-(1-Piperazinyl)verbindung (bei n = 1) der Formel (IV)

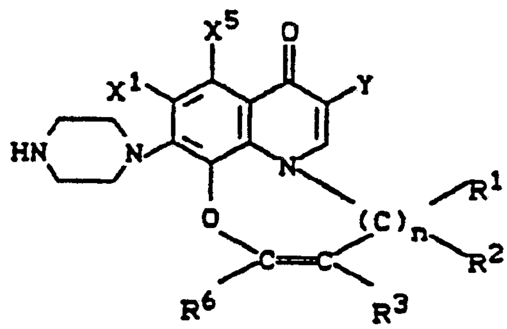

(IV)

in welcher
$X^1$, $X^5$, $R^1$, $R^2$, $R^3$ und $R^6$, Y sowie n die oben angegebenen Bedeutungen haben und der Piperazinylrest an den Kohlenstoffatomen in der bei $R^{10}$ und $R^{11}$ angegebenen Weise substituiert sein kann, mit Verbindungen der Formel (V)

$$R^{12}X \quad (V)$$

in welcher
$R^{12}$ die oben angegebene Bedeutung hat, jedoch nicht Wasserstoff sein kann und
X Fluor, Chlor, Brom, Iod, Hydroxy, Acyloxy, Ethoxy, Phenoxy, 4-Nitrophenoxy bedeutet, gegebenenfalls in Gegenwart von Säurebindern umsetzt (Methode B).

Bei dieser Reaktionstührung kann also der in der 10-bzw. 11-Stellung befindliche Piperazinylrest in einem ersten Reaktionsschritt nach der zuerst genannten Methode eingeführt werden - was zu der bereits erfindungsgemäßen Verbindung (IV) führt - und in einem Folgeschritt dann gewünschte weitere Substituenten eingeführt werden, hier beispielhaft $R^{12}$.

In einer weiteren Ausgestaltung des erfindungsgemäßen Verfahrens erhält man erfindungsgemäße Verbindungen der Formel (I), wenn man 10-(1-Piperazinyl) chinoloncarbonsäurederivate (n=0) bzw. 11-(1-Piperazinyl) chinoloncarbonsäurederivate (n=1) der Formel (IV), in welcher der Piperazinylrest an den Kohlenstoffatomen in der bereits genannten Weise substituiert sein kann, mit Michael-Acceptoren der Formel (VI),

$$B\text{–}CH{=}CH_2 \quad \text{(VI)}$$

in der

B für CN, CO-$R^{15}$ oder COOR$^{16}$ steht, wobei $R^{15}$ für Methyl oder Ethyl und $R^{16}$ für Methyl, Ethyl, n- oder i-Propyl steht, umsetzt (Methode C).

Verwendet man beispielsweise bei der Umsetzung nach der erstgenannten Methode 1-Methylpiperazin und 9,10-Difluor-7-oxo-2-phenyl-7H-pyrido [1,2,3-de][1,4]benzoxazin-6-carbonsäure als Ausgangsstoffe, so kann der Reaktionsablauf durch das folgende Formelschema wiedergegeben werden :

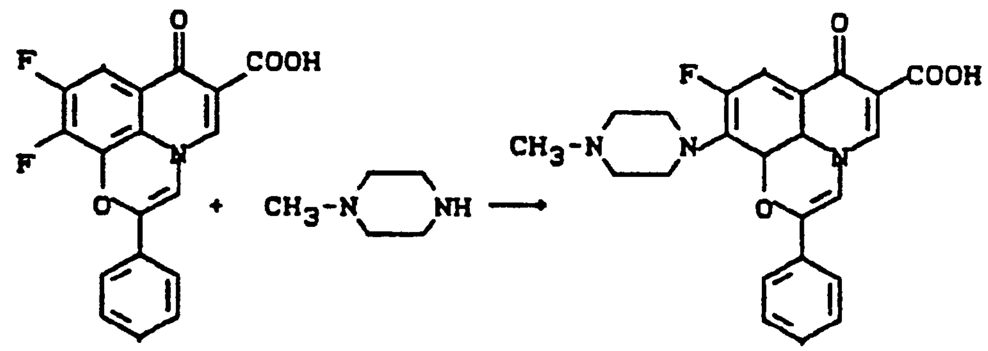

Verwendet man z. B. bei der Umsetzung nach der modifizierten Methode Ethyliodid und 9-Fluor-7-oxo-2-phenyl-10(1-piperazinyl)-7H-pyrido[1,2,3-de][1,4]benzoxazincarbonsäure als Ausgangsstoffe, so kann der Reaktionsablauf durch folgendes Formelschema wiedergegeben werden :

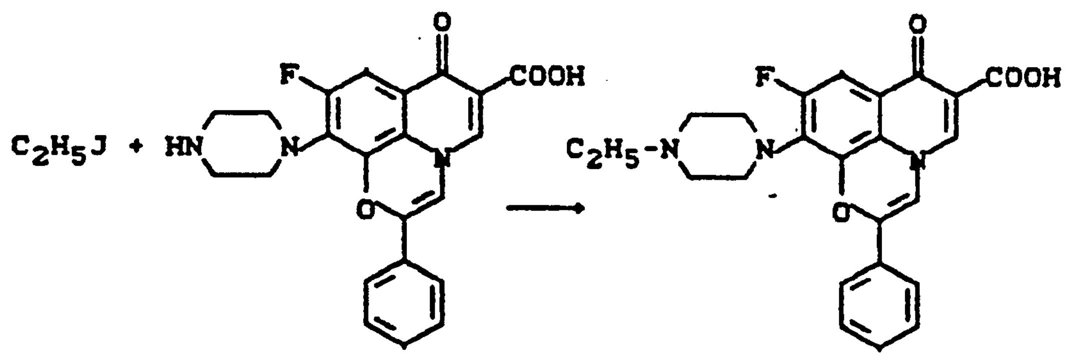

Die Umsetzung mit Michael-Acceptoren mit beispielsweise 9-Fluor-7-oxo-2-phenyl-10(1-piperazinyl)-7H-pyrido[1,2,3] [1,4]benzoxazin-6-carbonsäure und Methylvinylketon als Ausgangsstoffen kann durch folgendes Formelschema wiedergegeben werden :

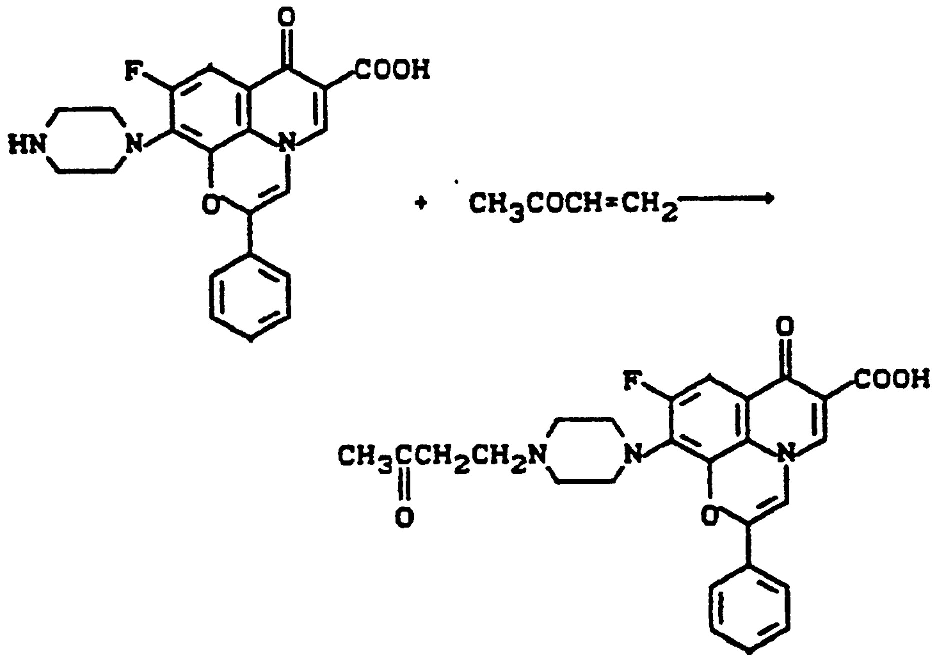


Die als Ausgangsstoffe im erfindungsgemäßen Verfahren verwendbaren Chinoloncarbonsäuren der Formel (II) können gemäß folgendem Schema hergestellt werden (Verfahren 1).

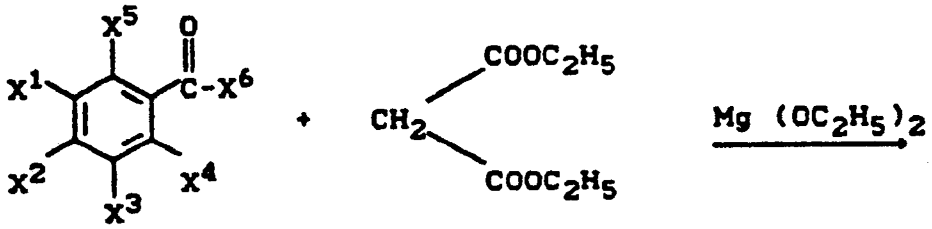


(1) (2)

$X^3 = X^4 = F, Cl, NO_2$ $X^6 = Cl, Br, F$

$X^2$, $X^3$, $X^5$ wie oben angegeben

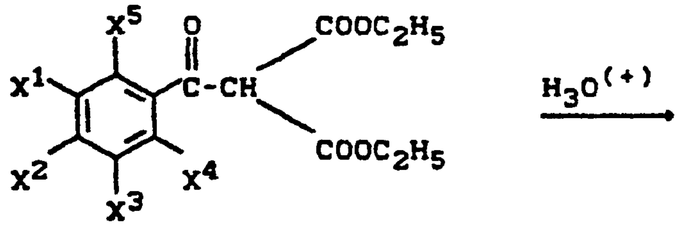


(3)

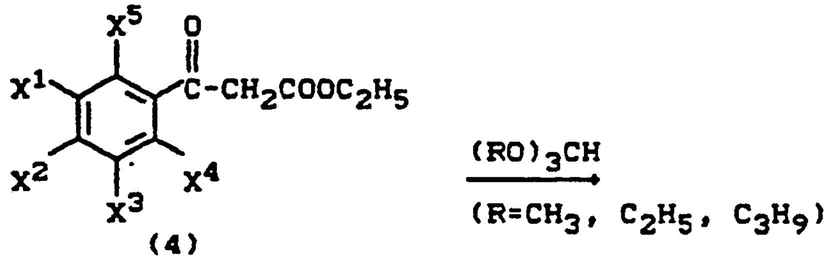


(4)

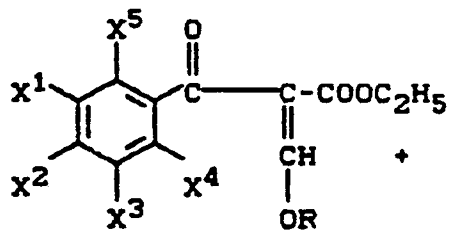

+

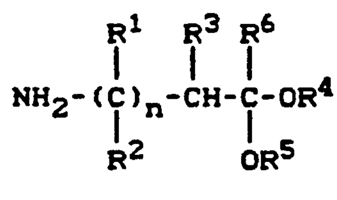

(6)

(5) $R^4$, $R^5$ = $CH_3$, $C_2H_5$,

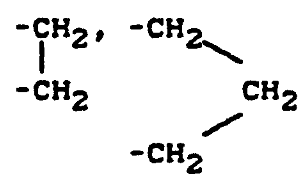

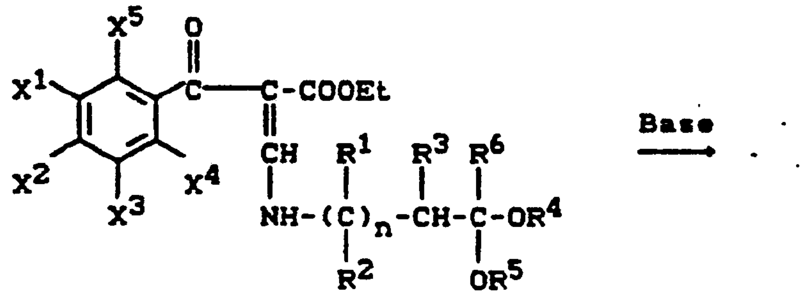


(7)

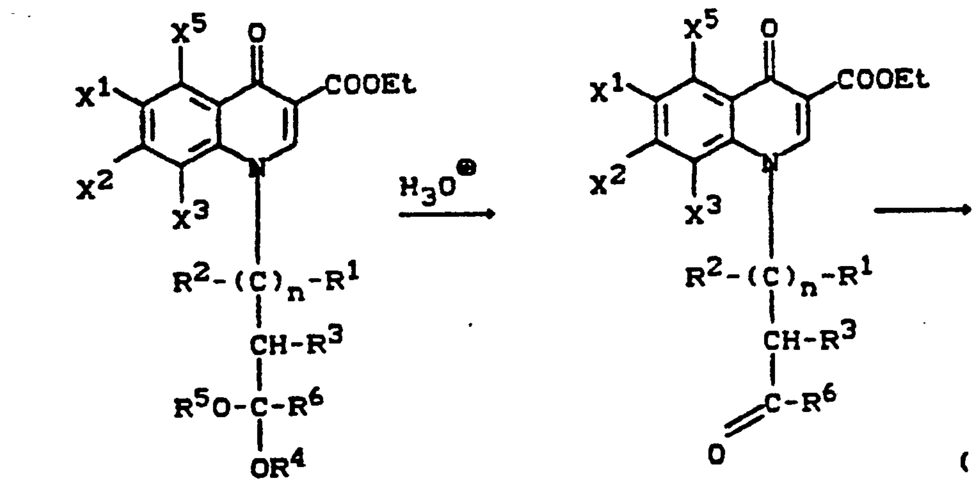


(9)

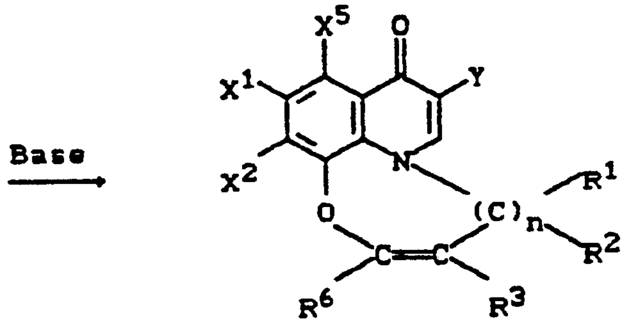


(II)

Danach wird Malonsäurediethylester (2) in Gegenwart von Magnesiumethylat mit dem entsprechenden Benzoylfluorid bzw. -chlorid (1) zum Benzoylmalonester (3) acyliert (Organicum, 3. Auflage 1964, S. 438).

Durch partielle Verseifung und Decarboxylierung von (3) in wäßrigem Medium mit katalytischen Mengen Schwefelsäure oder p-Toluolsulfonsäure erhält man in guter Ausbeute den Benzoylessigsäureethylester (4), der mit Orthoameisensäuretriethylester/Acetanhydrid in den 3-Ethoxyacrylsäureethylester (5) übergeht. Die Umsetzung von (5) mit dem entsprechenden Amin (6) in einem Lösungsmittel, wie z. B. Methylenchlorid, Alkohol, Chloroform, Cyclohexan oder Toluol führt in leicht exothermer Reaktion zum gewünschten Zwischenprodukt (7).

Die Cyclisierungsreaktionen (7) → (8) werden in einem Temperaturbereich von etwa 60 bis 300°C, bevorzugt 80 bis 180°C, durchgeführt.

Als Verdünnungsmittel können Dioxan, Dimethylsulfoxid, N-Methylpyrrolidon, Sulfolan, Hexamethylphosphorsäuretriamid und bevorzugt N,N-Dimethylformamid verwendet werden.

Als Säurebinder kommen für diese Reaktionsstute Kaliumtert.-butanolat, Butyl-lithium, Lithiumphenyl, Phenylmagnesiumbromid, Natriummethylat, Natriumhydrid, Natrium- oder Kaliumcarbonat und besonders bevorzugt Kalium- oder Natrium-fluorid in Betracht.

Die Hydrolyse des Acetals bzw. Ketals (8) unter Bildung von (9) erfolgt in saurer Lösung bevorzugt in wäßrig mineralsaurer Lösung.

Die im letzten Schritt erfolgende Cyclisierung von (9)→ (II) wird in einem Temperaturbereich von etwa 60-300°, bevorzugt 80-180°C durchgeführt.

Als Verdünnungsmittel können Alkohol, Dioxan, Dimethylsulfoxid, Hexamethylphosphorsäuretriamid und bevorzugt Wasser verwendet werden.

Als Säurebinder können für diese Reaktionsstufe Kaliumtert.-butanolat, Butyllithium, Phenyllithium, Phenylmagnesiumbromid, Natriummethylat, Natriumhydrid und bevorzugt Kalium- oder Natriumhydroxid in Betracht.

Die als Ausgangsstoffe für diesen Syntheseweg (Verfahren 1) verwendeten 2,3,4,5-Tetrafluorbenzoylchloride oder das Pentafluorbenzoylchlorid sind bekannt.

3,5-Dichlor-2,4-difluor-benzoylfluorid (Siedepunkt 97°/29 mbar ; $n^{20}_D$ = 1,5148) und 5-Chlor-2,3,4-tri-

fluorbenzoylfluorid (Siedepunkt 66-70° /20 mbar ; $n^{20}_D$=1,4764) erhält man nebeneinander beim Erhitzen von Tetrachlorbenzoylchlorid mit Kaliumfluorid in Sulfolan auf erhöhte Temperaturen :

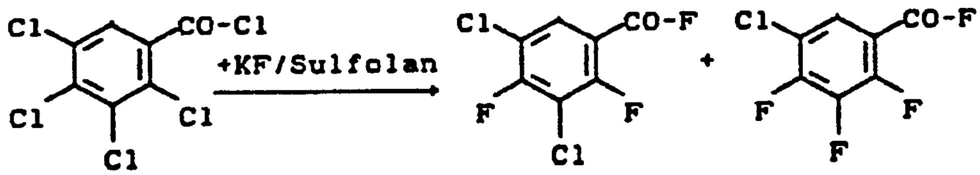


Die Chlorierung von 2,4,5-Trifluorbenzoesäure in Chlorsulfonsäure führt zur 3-Chlor-2,4,5-trifluorbenzoesäure, die als Rohprodukt mit Thionylchlorid zum 3-Chlor-2,4,5-trifluorbenzoylchlorid (Siedepunkt 94°/18 mbar ; $n^{20}_D$=1,5164) umgesetzt wird :

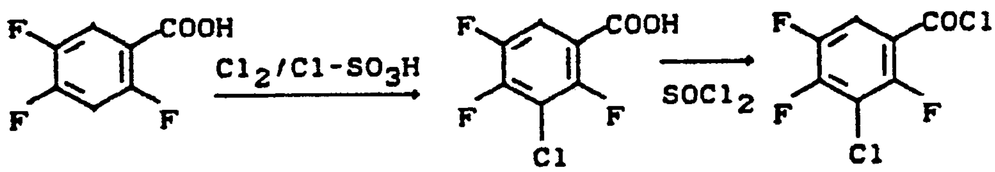


Das 2,4-Dichlor-5-fluor-3-nitro-benzoylchlorid wird durch Nitrierung der bekannten 2,4-Dichlor-5-fluorbenzoesäure zur 2,4-Dichlor-5-fluor-3-nitro-benzoesäure und deren Umsetzung mit Thionylchlord erhalten.

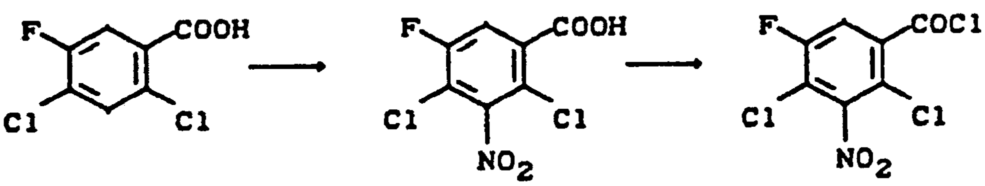

Die als Ausgangsstoffe verwendeten Amine der Formel (6) sind bekannt. Als Beispiele seien genannt: Aminoacetaldehyd-dimethylacetal, Aminoacetaldehyd-diethylacetal, 2-Amino-propionaldelhyd-dimethylacetal, 2-Aminocyclohexanon-dimethylketal, 2-Aminomethyl-1,3-dioxan, 2-Aminomethyl-1,3-dioxan, 2-(1-Amino-1-ethyl)-1,3-dioxan.

Die als Ausgangsstoffe nach Methode A verwendbaren Chinoloncarbonsäuren der Formel II können im Fall n=Q, ebenfalls gemäß folgendem Schema erhalten werden (Verfahren 2).

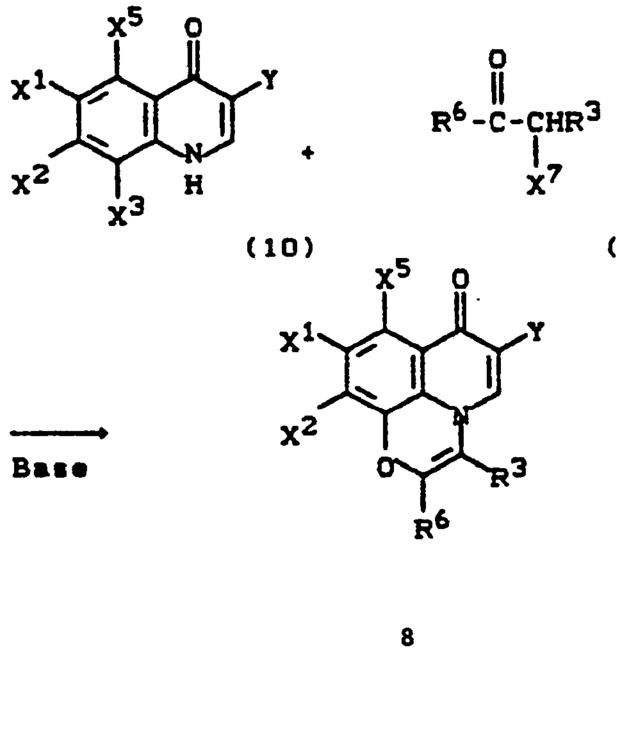


(10) (11) $X^7$ = Cl, Br

II (n = o)

Demnach wird ein 4-Chinolon-3-carbonsäurederivat (10) in dem $X^1$, $X^2$, $X^3$ und Y die oben angegebene Bedeutung haben mit einer α-Halogencarbonylverbindung 11 in der $R^3$ und $R^6$ die oben angegebene Bedeutung haben in Gegenwart einer Base umgesetzt, wobei Alkylierung und Ringschluß zu II in einem Schritt in einer sogenannten Eintopfreaktion erfolgen.

Die Reaktion wird in einem Temperaturbereich von 60 bis 300°C, bevorzugt 80 bis 180°C durchgeführt.

Als Verdünnungsmittel können Dioxan, Dimethylsulfoxid N-Methylpyrrolidon, Sulfolan, Hexamethylphosphorsäuretriamid und bevorzugt N,N-Dimethylformamid verwendet werden.

Als Säurebinder kommen für diese Reaktionsstufe Kaliumtert.-butanolat, Butyllithium, Phenyllithium, Phenylmagnesiumbromid, Natriummethylat, Natriumhydrid und bevorzugt Natrium- bzw. Kaliumcarbonat in Betracht.

Die als Ausgangsstoffe für diesen Syntheseweg (Verfahren 2) verwendeten 4-Chinolon-3-carbonsäurederivate bzw. 4-Hydroxy-3-chinolincarbonsäurederivate der Formel (10) sind bekannt. Als Beispiel seien genannt:

7-Chlor-6,8-difluor-4-hydroxy-5-nitro-3-chinolincarbonsäureethylester
6,7,8-Trifluor-4-hydroxy-3-chinolincarbonsäureethylester.

Die als Ausgangsstoffe verwendeten α-Halogencarbonylverbindungen sind bekannt. Als Beispiele seien genannt:

Chloracetaldehyd, Chloraceton, ω-Bromacetophenon, 2-Chlorcyclohexanon, 2-Chlorbutanon, 1,3-Dichloraceton.

Beispiel für eine erfindungsgemäße Tablette

Jede Tablette enthält:

| | |
|---|---|
| Verbindung des Beispiels 1 | 583,0 mg |
| Mikrokristalline Cellulose | 55,0 mg |
| Maisstärke | 72,0 mg |
| Poly-(1-vinyl-2-pyrrolidon) unlöslich | 30,0 mg |
| Hochdisperses Siliciumdioxid | 5,0 mg |
| Magnesiumstearat | 5,0 mg |
| | 750,0 mg |

Die Lackhülle enthält:

| | |
|---|---|
| Poly-(O-hydroxypropyl-O-methyl)-cellulose 15 cp | 6,0 mg |
| Macrogol 4000 rec. INN Polyethylenglykole (DAB) | 2,0 mg |
| Titan-(IV)-oxid | 2,0 mg |
| | 10,0 mg |

Die erfindungsgemäßen Verbindungen zeigen bei geringer Toxizität ein breites antibakterielles Spektrum gegen gram-positive und gram-negative Keime, insbesondere gegen Enterobakteriaceen ; vor allem auch gegen solche, die resistent sind gegen verschiedene Antibiotika, wie z.B. Penicilline, Cephalosporine, Aminoglykoside, Sulfonamide, Tetracycline.

Diese wertvollen Eigenschaften ermöglichen ihre Verwendung als chemotherapeutische Wirkstoffe in der Medizin sowie als Stoffe zur Konservierung von anorganischen und organischen Materialien, insbesondere von organischen Materialien aller Art, z.B. Polymeren, Schmiermitteln, Farben, Fasern, Leder, Papier und Holz, von Lebensmitteln und von Wasser.

Die erfindungsgemäßen Verbindungen sind gegen ein sehr breites Spektrum von Mikroorganismen wirksam. Mit ihrer Hilfe können gram-negative und gram-positive Bakterien und bakterienähnliche Mikroorganismen bekämpft sowie die durch diese Erreger hervorgerufenen Erkrankungen verhindert, gebessert und/oder geheilt werden,

Besonders wirksam sind die erfindungsgemäßen Verbindungen gegen Bakterien und bakterienähnliche Mikroorganismen. Sie sind daher besonders gut zur Prophylaxe und Chemotherapie von lokalen und systemischen Infektionen in der Human- und Tiermedizin geeignet, die durch diese Erreger hervorgerufen werden.

Beispielsweise können lokale und/oder systemische Erkrankungen behandelt und/oder verhindert werden, die durch die folgenden Erreger oder durch Mischungen der folgenden Erreger verursacht werden :

Gram-positive Kokken, z.B. Staphylokokken (Staph. aureus, Staph. epidermidis) und Streptokokken (Strept. agalactiae, Strept. faecalis, Strept. pneumoniae, Strept. pyogenes) ; gram-negative Kokken (Neisseria gonorrhoeae) sowie gram-negative Stäbchen wie Enterobakteriaceen, z.B. Escherichia coli, Hämophilus influenzae, Citrobacter (Citrob. freundii, Citrob. divernis), Salmonella und Shigella ; ferner Klebsiellen (Klebs. pneumoniae, Klebs. oxytoca), Enterobacter (Ent. aerogenes, Ent. agglomerans), Hafnia, Serratia (Serr. marcescens), Proteus (Pr, mirabilis, Pr. rettgeri, Pr. vulgaris), Providencia, Yersinia, sowie die Gattung Acinetobacter. Darüber hinaus umfaßt das antibakterielle Spektrum die Gattung Pseudomonas (Ps, aeruginosa, Ps. maltophilia) sowie strikt anaerobe Bakterien wie z,B, Bacteroides fragilis, Vertreter der Gattung Peptococcus, Peptostreptococcus sowie die Gattung Clostridium ; ferner Mykoplasmen (M, pneumoniae, M, hominis, M, urealyticum) sowie Mykobakterien, z.B. Mycobacterium tuberculosis.

Die obige Aufzählung von Erregern ist lediglich beispielhaft und keineswegs beschränkend aufzufassen. Als Krankheiten, die durch die genannten Erreger oder Mischinfektionen verursacht und durch die erfindungsgemäßen Verbindungen verhindert, gebessert oder geheilt werden können, seien beispielsweise genannt :

Infektionskrankheiten beim Menschen wie zum Beispiel Otitis, Pharyngitis, Pneumonie, Peritonitis, Pyelonephritis, Cystitis, Endocarditis, Systeminfektionen, Bronchitis (akut, chronisch), septische Infektionen, Erkrankungen der oberen Luftwege, diffuse Panbronchiolitis, pulmonäres Emphysem, Dysenterie, Enteritis, Leberabszesse, Urethritis, Prostatitis, Epididymitis, gastrointestinale Infektionen, Knochen- und Gelenkinfektionen, zystische Fibrose, Hautinfektionen, postoperative Wundinfektionen, Abszesse, Phlegmone, Wundinfektionen, infizierte Verbrennungen, Brandwunden, Infektionen im Mundbereich, Infektionen nach Zahnoperationen, Osteomyelitis, septische Arthritis, Cholecystitis, Peritonitis mit Appendicitis, Cholangitis, intraabdominale Abszesse, Pankreatitis, Sinusitis, Mastoiditis, Mastitis, Tonsillitis, Typhus, Meningitis und Infektionen des Nervensystems, Salpingitis, Endometritis, Genital-Infektionen, Pelveoperitonitis und Augeninfektionen.

Außer beim Menschen können bakterielle Infektionen auch bei anderen Spezies behandelt werden. Beispielhaft seien genannt :

Schwein : Coli-diarrhoe, Enterotoxämie, Sepsis, Dysenterie, Salmonellose, Metritis-Mastitis-Agalaktiae-Syndrom, Mastitis ;

Wiederkäuer (Rind, Schaf, Ziege) : Diarrhoe, Sepsis, Bronchopneumonie, Salmonellose, Pasteurellose, Mykoplasmose, Genitalinfektionen ;

Pferd : Bronchopneumonien, Fohlenlähme, puerperale und postpuerperale Infektionen, Salmonellose;

Hund und Katze : Bronchopneumonie, Diarrhoe, Dermatitis, Otitis, Harnwegsinfekte, Prostatitis ;

Geflügel (Huhn, Pute, Wachtel, Taube, Ziervögel und andere) : Mycoplasmose, E, coli-Infektionen, chronische Luftwegserkrankungen, Salmonellose, Pasteurellose, Psittakose.

Ebenso können bakterielle Erkrankungen bei der Aufzucht und Haltung von Nutz- und Zierfischen behandelt werden, wobei sich das antibakterielle Spektrum über die vorher genannten Erreger hinaus auf weitere Erreger wie zum Beispiel Pasteurella, Brucella, Campylobacter, Listeria, Erysipelothrix, Corynebakterien, Borellia, Treponema, Nocardia, Rikettsien, Yersinia, erweitert.

Zur vorliegenden Erfindung gehören pharmazeutische Zubereitungen, die neben nicht-toxischen, inerten pharmazeutisch geeigneten Trägerstoffen eine oder mehrere erfindungsgemäße Verbindungen enthalten oder die aus einem oder mehreren erfindungsgemäßen Wirkstoffen bestehen, sowie Verfahren zur Herstellung dieser Zubereitungen.

Zur vorliegenden Erfindung gehören auch pharmazeutische Zubereitungen in Dosierungseinheiten. Dies bedeutet, daß die Zubereitung in Form einzelner Teile, z.B, Tabletten, Dragees, Kapseln, Pillen, Suppositorien und Ampullen vorliegen, deren Wirkstoffgehalt einem Bruchteil oder einem Vielfachen einer Einzeldosis entspricht. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem

Viertel einer Tagesdosis entspricht.

Unter nicht-toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Tabletten, Dragees, Kapseln, Pillen und Granulate können den oder die Wirkstoffe neben den üblichen Trägerstoffen enthalten, wie (a) Füll- und Streckmittel, z.B. Stärken, Milchzucker, Rohrzucker, Glukose, Mannit und Kieselsäure, (b) Bindemittel, z.B. Carboxymethylcellulose, Alginate, Gelatine, Polyvinylpyrrolidon, (c) Feuchthaltemittel, z.B. Glycerin, (d) Sprengmittel, z.B. Agar-Agar, Calciumcarbonat und Natriumcarbonat, (e) Lösungsverzögerer, z.B. Paraffin und (f) Resorptionabeschleuniger, z.B. quarternäre Ammoniumverbindungen, (g) Netzmittel, z.B. Cetylalkohol, Glycerinmonostearat, (h) Adsorptionsmittel, z.B. Kaolin und Bentonit und (i) Gleitmittel z.B. Talkum, Calcium- und Magnesiumstearat und feste Polyethylenglykole oder Gemische der unter (a) bis (i) aufgeführten Stoffe.

Die Tabletten, Dragees, Kapseln, Pillen und Granulate können mit den üblichen, gegebenenfalls Opakisierungsmitteln enthaltenden, Überzügen und Hüllen versehen sein und auch so zusammengesetzt sein, daß sie den oder die Wirkstoffe nur oder bevorzugt in einem bestimmten Teil des Intestinaltraktes gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z.B. Polyethylenglykole, Fette, z.B. Kakaofett und höhere Ester (z.B. $C_{14}$-Alkohol mit $C_{16}$-Fettsäure) oder Gemische dieser Stoffe.

Salben, Pasten, Cremes und Gele können neben dem oder den Wirkstoffen die üblichen Trägerstoffe enthalten z.B. tierische und pflanzliche Fette, Wachse, Paraffine, Stärke, Tragant, Cellulosederivate, Polyethylenglykole, Silikone, Bentonite, Kieselsäure, Talkum und Zinkoxid oder Gemische dieser Stoffe.

Puder und Sprays können neben dem oder den Wirkstoffen die üblichen Trägerstoffe enthalten, z.B. Milchzucker, Talkum, Kieselsäure, Aluminiumhydroxid, Calciumsilikat und Polyamidpulver oder Gemische dieser Stoffe. Sprays können zusätzlich die üblichen Treibmittel, z.B. Chlorfluorkohlenwasserstoffe, enthalten.

Lösungen und Emulsionen können neben dem oder den Wirkstoffen die üblichen Trägerstoffe wie Lösungsmittel, Lösungsvermittler und Emulgatoren z.B. Wasser, Ethylalkohol Isopropylalkohol Ethylcarbonat Ethylacetat Benzylalkohol Benzylbenzoat Propylenglykol 1,3-Butylenglykol Dimethylformamid, Öle, insbesondere Baumwollsaatöl, Erdnußöl, Maiskeimöl, Olivenöl, Ricinusöl und Sesamöl, Glycerin, Glycerinformal, Tetrahydofurfurylalkohol Polyethylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel, z.B. Wasser, Ethylalkohol, Propylenglykol, Suspendiermittel, z.B. Ethoxylierte Isostearylalkohole, Polyoxyethylensorbit- und Sorbitan-Ester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmacksverbessernde Zusätze z.B. Pfefferminzöl und Eukalyptusöl und Süßmittel z.B. Saccharin, enthalten.

Die therapeutisch wirksamen Verbindungen sollen in den oben aufgeführten pharmazeutischen Zubereitungen vorzugsweise in einer Konzentration von etwa 0,1 bis 99,5 vorzugsweise von etwa 05 bis 95 Gew.-%, der Gesamtmischung vorhanden sein.

Die oben aufgeführten pharmazeutischen Zubereitungen können außer den erfindungsgemäßen Verbindungen auch weitere pharmazeutische Wirkstoffe enthalten.

Die Herstellung der oben aufgeführten pharmazeutischen Zubereitungen erfolgt in üblicher Weise nach bekannten Methoden z.B. durch Mischen des oder der Wirkstoffe mit dem oder den Trägerstoffen.

Die genannten Zubereitungen können bei Mensch und Tier entweder oral, rektal, parenteral (intravenös, intramuskulär, subkutan), intracisternal, intravaginal, intraperitoneal, lokal (Puder, Salbe, Tropfen) und zur Therapie von Infektionen in Hohlräumen, Körperhöhlen angewendet werden. Als geeignete Zubereitungen kommen Injektionlösungen, Lösungen und Suspensionen für die orale Therapie, Gele, Aufgußformulierungen, Emulsionen, Salben oder Tropfen in Frage. Zur lokalen Therapie können ophtalmologische und

dermatologische Formulierungen, Silber- und andere Salze, Ohrentropfen, Augensalben, Puder oder Lösungen verwendet werden. Bei Tieren kann die Aufnahme auch über das Futter oder Trinkwasser in geeigneten Formulierungen erfolgen. Ferner können Gele, Pulver, Puder, Retard-Tabletten, Premixe, Konzentrate, Granulate, Pellets, Tabletten, Boli, Kapseln, Aerosole, Sprays, Inhalate bei Mensch und Tier angewendet werden. Ferner können die erfindungsgemäßen Verbindungen in andere Trägermaterialien wie zum Beispiel Kunststoffe (Kunststoffketten zur lokalen Therapie), Kollagen oder Knochenzement eingearbeitet werden.

Im allgemeinen hat es sich sowohl in der Human- als auch in der Veterinärmedizin als vorteilhaft erwiesen, den oder die erfindungsgemäßen Wirkstoffe in Gesamtmengen von etwa 0,5 bis etwa 500, vorzugsweise 5 bis 100 mg/kg Körpergewicht je 24 Stunden, gegebenenfalls in Form mehrerer Einzelgaben, zur Erzielung der gewünschten Ergebnisse zu verabreichen, Eine Einzelgabe enthält den oder die erfindungsgemäßen Wirkstoffe vorzugsweise in Mengen von etwa 1 bis etwa 80, insbesondere 3 bis 30 mg/kg Körpergewicht. Es kann jedoch erforderlich sein von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt.

So kann es in einigen Fällen ausreichend sein, mit weniger als der obengenannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Die neuen Verbindungen können in den üblichen Konzentrationen und Zubereitungen zusammen mit dem Futter bzw, mit Futterzubereitungen oder mit dem Trinkwasser gegeben werden. Dadurch kann eine Infektion durch gram-negative oder gram-positive Bakterien verhindert, gebessert und/oder geheilt werden und dadurch eine Förderung des Wachstums und eine Verbesserung der Verwertung des Futters erreicht werden.

Die folgenden Beispiele erläutern die Erfindung.

Beispiel 1

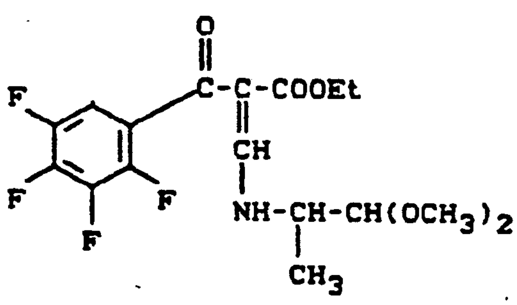

6,4 g 3-Ethoxy-2-(2,3,4,5-tetrafluorbenzoyl)-acrylsäureethylester werden in 8 ml Ethanol vorgelegt. Unter Eiskühlung wird eine Lösung von 26 g 2-Amino-propionaldehyddimethylacetal in 15 ml Ethanol zugetropft. Man läßt auf Raumtemperatur kommen, rührt zwei Stunden und engt dann im Vakuum ein. Ausbeute 8 g 2-(2,3,4,5-Tetrafluorbenzoyl)-3-(1-methyl-2,2-dimethoxy-1-methyl-ethylamino)-acrylsäureethylester (Rohprodukt, Öl).

Beispiel 2

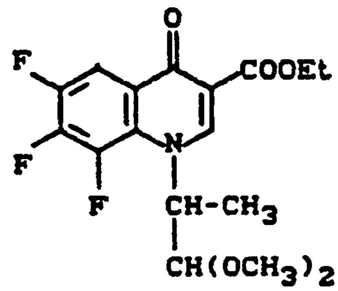

64 g Rohprodukt aus Beispiel 1 und 24 g $K_2CO_3$ werden in 370 ml DMF vier Stunden auf 140°C erhitzt. Anschließend gießt man auf Eis. Der Niederschlag wird in $CH_2Cl_2$ aufgenommen. Die organische Phase wird gewaschen und über $Na_2SO_4$ getrocknet. Nach Einengen im Vakuum werden 50 g eines schmierigen Feststoffes erhalten, der durch Waschen mit Ether gereinigt wird, Ausbeute 42 g 6,7,8Trifluor-1,4-dihydro-1-(2,2-dimethoxy-1-methyl-ethyl)-4-oxo-3-chinolincarbonsäureethylester.
Schmelzpunkt : 125-6°C.

Beispiel 3

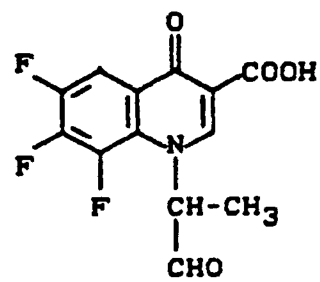

4,4 g Produkt aus Beispiel 2 werden zusammen mit 15 ml Essigsäure, 13 ml Wasser und 1,3 ml Schwetelsäure vier Stunden auf 140°C erhitzt. Nach Abkühlung auf Raumtemperatur gibt man Wasser zu und isoliert den ausgefallenen Feststoff. Er ist für weitere Umsetzungen rein genug. Ausbeute 3,0 g 6,7,8-Trifluor-1,4-dihydro-4-oxo-1-(1-oxo-2-propyl)-3-chinolincarbonsäure.
Schmelzpunkt 188-90°C (Z).

Beispiel 4

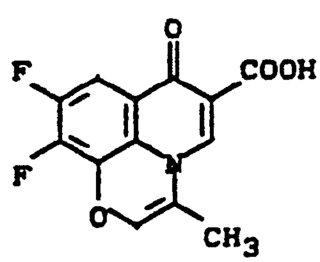

2,2 g Produkt aus Beispiel 3 werden mit 0,6 g NaOH in einem Gemisch aus 20 ml Wasser und 20 ml Ethanol vier Stunden gekocht. Nach Abkühlung auf Raumtemperatur wird mit verdünnter Salzsäure angesäuert und der entstandene Feststoff isoliert.

Ausbeute 1,8 g 9,10-Difluor-3-methyl-7-oxo-7H -pyrido[1,2,3 de]-[1,4]benzoxazin-6-carbonsäure.
Schmelzpunkt 248-50°C (Z).

Beispiel 5

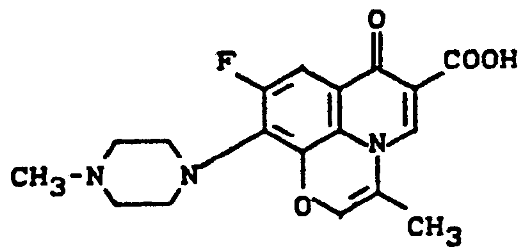

1,6g Produkt aus Beispiel 4 und 2,9 g N-Methyl-piperazin werden in 20 ml DMSO 2,5 Stunden auf 140°C erhitzt. Nach Abkühlung wird im Hochvakuum eingeengt. Der Rückstand wird mit Wasser versetzt. Den Niederschlag isoliert man und trocknet ihn im Vakuum bei 50°C.

Ausbeute 0,4 g 9-Fluor-3-methyl-10-(4-methyl-1-piperazinyl)-7-oxo-7H pyrido[1,2,3 de][1,4]benzoxazin-6-carbonsäure.
Schmelzpunkt 262-3°C.

Beispiel 6

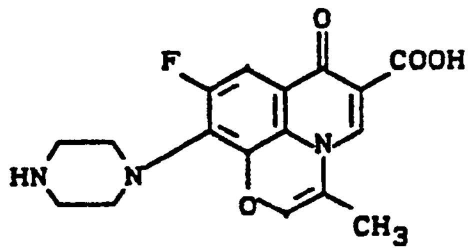

Analog zu Beispiel 5 wird 9-Fluor-3-methyl-10(1-piperazinyl)-7-oxo-7H-pyrido [1,2,3 de][1,4]benzoxazin-6-carbonsäure erhalten.
Schmelzpunkt 270-2°C (Z).

Beispiel 7

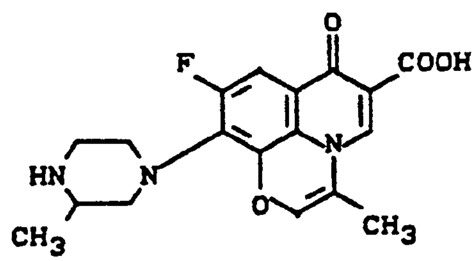

Analog zu Beispiel 5 wird 9-Fluor-3-methyl-10-(3-methyl-1-piperazinyl)-7-oxo-7H-pyrido[1,2,3 de][1,4]benzoxazin-6-carbonsäure erhalten.
Schmelzpunkt 190-4°C (Z).

Beispiel 8

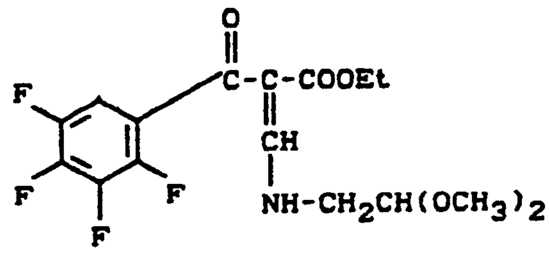

Analog zu Beispiel 1 wird durch Umsetzung von 3-Ethoxy-2-(2,3,4,5-tetrafluorbenzoyl)-acrylsäureethylester mit Aminoacetaldehyd-dimethylacetal der 2-(2,3,4,5-Tetrafluorbenzoyl)-3-(2,2-dimethoxy-ethylamino)acrylsäureethylester erhalten (Öl).

Beispiel 9

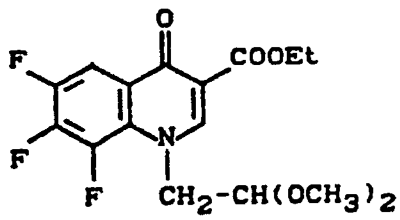

Die Cyclisierung des Produktes aus Beispiel 8 analog der Umsetzung in Beispiel 2 ergibt 6,7,8-Trifluor-1,4-dihydro-1-(2,2-dimethoxyethyl)-4-oxo-3-chinolincarbonsäureethylester.
Schmelzpunkt 104-6°C.

Beispiel 10

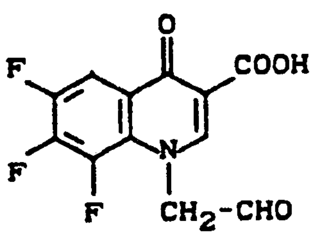

Die Verseifung des Produktes aus Beispiel 9 analog der Umsetzungin Beispiel 3 ergibt 6,7,8-Trifluor-1,4-dihydro-1-(2-oxo-1-ethyl)-3-chinolincarbonsäure.
Schmelzpunkt 220-2° C.

Beispiel 11

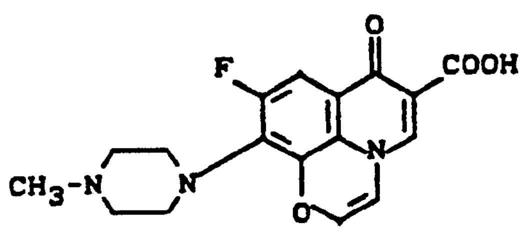

2,85 g Produkt aus Beispiel 10 und 5,0 g N-Methylpiperazin werden in 30 ml DMSO 2,5 Stunden auf

140°C erhitzt. Anschließend wird alles Flüchtige im Hochvakuum entfernt. Der Rückstand wird mit Acetonitril verrührt, Der Feststoff wird isoliert und getrocknet. Ausbeute 2,6 g 9-Fluor-10-(4-methyl-1-piperazinyl)-7-oxo-7H-pyrido [1,2,3 de][1,4]benzoxazin-6-carbonsäure.
Schmelzpunkt > 300°C.

Beispiel 12

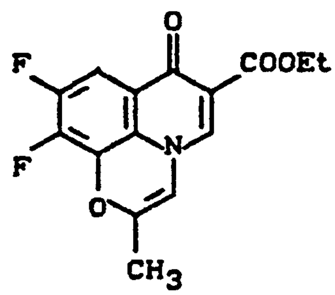

5 g 6,7,8-Trifluor-4-hydroxy-3-chinolincarbonsäureethylester, 3,5 g Chloraceton und 6,6 g $K_2CO_3$ werden in 40 ml DMF zehn Stunden auf 90°C erhitzt. Nach Abkühlen wird im Vakuum alles Flüchtige abgezogen, Der Rückstand wird mit Wasser verrührt. Der zurückbleibende Feststoff wird isoliert, getrocknet und aus DMF umkristallisiert. Ausbeute 3,4 g 9,10-Difluor-2-methyl-7-oxo-7H-pyrido [1,2,3 de][1,4]benzoxazin-6-carbonsäureethylester.
Schmelzpunkt 229-30° C.

Beispiel 13

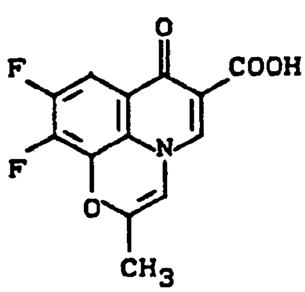

2,6 g Produkt aus Beispiel 12 werden zusammen mit 10 ml Essigsäure, 8 ml Wasser und 0,9 ml $H_2SO_4$ vier Stunden auf 140°C erhitzt. Nach Abkühlen wird mit Wasser verdünnt. Der Niederschlag wird isoliert und getrocknet. Ausbeute 2,4 g 9,10-Difluor-2-methyl-7-oxo-7H-pyrido [1,2,3 de][1,4]benzoxazin-6-carbonsäure.
Schmelzpunkt > 300°C.

Beispiel 14

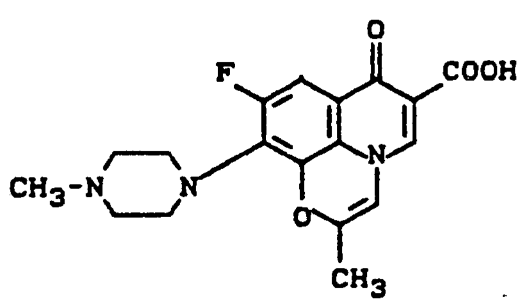

2,4 g Produkt aus Beispiel 13 und 4,3 g N-Methylpiperazin werden in 25 ml DMSO 2,5 Stunden auf 140°C erhitzt. Anschließend wird alles Flüchtige im Vakuum abgezogen, Der Rückstand wird mit Wasser verrührt. Der verbleibende Feststoff wird isoliert und getrocknet.

Ausbeute 1,3 g 9-Fluor-2-methyl-10(4-methyl-1-piperazinyl)-7-oxo-7H-pyrido[1,2,3 de][1,4]benzoxazin-6-carbonsäure.
Schmelzpunkt 255-6°C (Z).

Beispiel 15

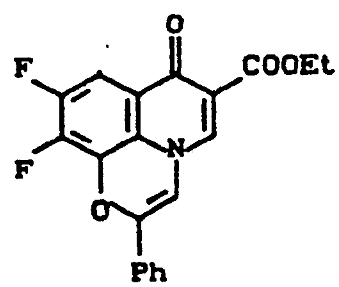

5 g 67,8-Trifluor-4-hydroxy-3-chinolincarbonsäureethylester 5,9 g ω-Chloracetophenon und 6,6 g $K_2CO_3$ werden in 40 ml DMF 10 Stunden auf 90°C erhitzt. Anschließend wird im Vakuum eingeengt. Den Rückstand verrührt man mit Wasser, isoliert den Feststoff und kristallisiert ihn aus DMF um.

Ausbeute 2,4 g 9,10-Difluor-7-oxo-2-phenyl-7H-pyrido [1,2,3 de][1,4]benzoxazin-6-carbonsäureethylester.
Schmelzpunkt 251-52°C.

Beispiel 16

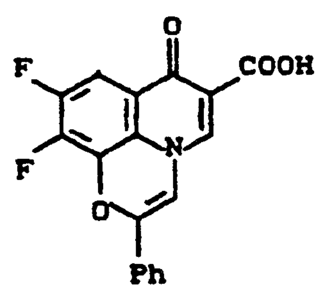

1,9g Produkt aus Beispiel 15, 6 ml Essigsäure, 4 ml Wasser und 0,5 ml Schwefelsäure werden 4 Stunden gekocht. Danach wird mit Wasser verdünnt und der Feststoff wird abgetrennt.

Ausbeute 1,7 g 9,10-Difluor-7-oxo-2-phenyl-7H-pyrido [1,2,3 de][1,4]benzoxazin-6-carbonsäure.
Schmelzpunkt >300°C.

Beispiel 17

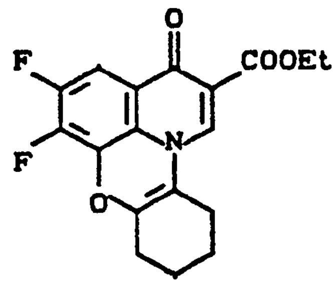

5 g 6,7,8-Trifluor-4-hydroxy-3-chinolincarbonsäureethylester, 5 g 2-Chlorcyclohexanon und 6,6 g $K_2CO_3$ werden in 40 ml DMF 10 Stunden auf 90°C erhitzt. Danach engt man im Vakuum ein. Der Rückstand wird in Wasser aufgenommen und mit $CH_2Cl_2$ extrahiert. Die organische Phase wird getrocknet und eingedampft. Den Rückstand trennt man mit Hilfe einer Säule (Kieselgel, Laufmittel : Toluol/Essigester 1 :1).

Ausbeute 0,6 g 6,7-Difluor-9-oxo-9H-cyclohexa[1,2-b] pyrido-[1',2',3'-de]1,4-benzoxazin-10-carbonsäureethylester.
Schmelzpunkt 198-208°C.

## Ansprüche

1. 1,8-verbrückte 4-Chinolon-3-carbonsäuren und Derivate der Formel (I)

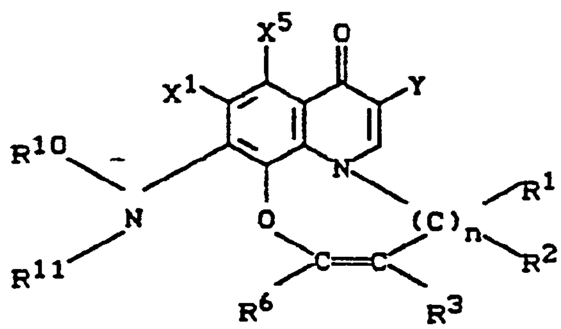

(I)

in welcher

Y für eine Carboxylgruppe steht,

$X^1$ für Fluor steht,

$X^5$ für Wasserstoff steht,

$R^{10}$ und $R^{11}$ gemeinsam mit dem Stickstoffatom, an das sie gebunden sind, einen 5- oder 6-gliedrigen heterocyclischen Ring bilden können, der als Ringglied zusätzlich ein Sauerstoffatom oder die Gruppe N-$R^{12}$enthalten kann und dergegebenenfalls an den Kohlenstoffatomen ein- bis zweifach durch $C_1$-$C_2$-Alkyl, Cyclohexyl, gegebenenfalls durch Chlor, Fluor, Methyl, Phenyl, Hydroxy, Methoxy, Benzyloxy, Nitro oder Piperidino substituiertes Phenyl, 2-Thienyl oder Hydroxy substituiert sein kann, wobei $R^{12}$ für Wasserstoff, eine verzweigte oder unverzweigte Alkylgruppe mit 1 bis 3 Kohlenstoffatomen, die gegebenenfalls durch eine oder zwei Hydroxygruppen substituiert sein kann, einen Phenacylrest, einen Oxalkylrest mit bis zu 4 Kohlenstoffatomen sowie einen Rest $COR^{13}$ steht, wobei $R^{13}$ Wasserstoff oder Alkyl mit ein oder zwei Kohlenstoffatomen bedeutet,

$R_1$, $R_2$, $R_3$ und R6 für Wasserstoff, eine Alkylgruppe mit vorzugsweise 1 - 6 Kohlenstoffatomen steht, sowie $R_6$ auch für Phenyl stehen kann und

n 0 oder 1 bedeutet

sowie die Verbindung 6,7-Difluor-9-oxo-9H-cyclohexa-[1,2-b]-pyrido-[1',2',3'-de]1,4-benzoxacin-10-carbonsäureethylester

und deren pharmazeutisch verwendbare Hydrate oder Salze, vorzugsweise Alkali-, Erdalkali-, Silber- und Guanidiniumsalze, sowie ihre Ester.

2. Verfahren zur Herstellung von 1,8-verbrückten 4-Chinolon-3-carbonsäuren und derivaten der Formel (I) gemäß Anspruch 1, dadurch gekennzeichnet, daß man Chinoloncarbonsäurederivate der Formel (II)

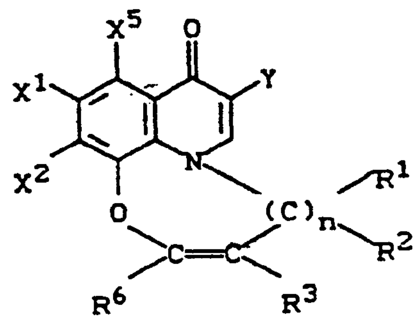

(II)

in der die Reste $X^1$, $X^5$, $R^1$, $R^2$, $R^3$ und $R^6$, Y sowie n die oben angegebenen Bedeutungen haben, sowie $X^2$ für Halogen, bevorzugt für Chlor und Fluor, steht,
mit-Aminen der Formel (III)

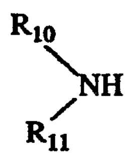

(III)

in welcher

$R^{10}$ und $R^{11}$ die oben angegebenen Bedeutungen haben, gegebenenfalls in Gegenwart von Säurebindern umsetzt.

Verfahren zur Herstellung von Verbindungen der Formel (I) gemäß Anspruch 1, dadurch gekennzeichnet, daß man eine 10-(1-Piperazinyl)verbindung (bei n = 0) bzw. 11-(1-Piperazinyl)verbindung (bei n = 1) der Formel (IV)

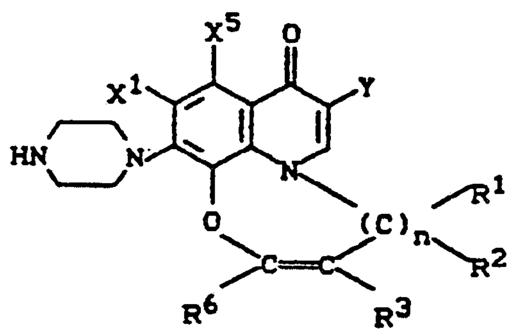

(IV)

in welcher

$X^1$, $X^5$, $R^1$, $R^2$, $R^3$ und $R^6$, Y sowie n die oben angegebenen Bedeutungen haben und der Piperazinylrest an den Kohlenstoffatomen in der bei $R^{10}$ und $R^{11}$ angegebenen Weise substituiert sein kann,
mit Verbindungen der Formel (V)

$R^{12}X$ (V)

in welcher

$R^{12}$ die oben angegebene Bedeutung hat, jedoch nicht Wasserstoff sein kann, und

X Fluor, Chlor, Iod, Hydroxy, Acyloxy, Ethoxy, Phenoxy, 4-Nitrophenoxy bedeutet, gegebenenfalls in Gegenwart von Säurebindern umsetzt.

4.Verfahren zur Herstellung von Verbindungen der Formel (I) gemäß Anspruch 1, dadurch gekennzeichnet, daß man 10(1-Piperazinyl)chinoloncarbonsäurederivate (n = 0) bzw. 11-(1-Piperazinyl)chinoloncarbonsäurederivate (n = 1) der Formel (IV) nach Anspruch 3, in welcher der Piperazinylrest an den Kohlenstoffatomen in der in Anspruch 3 genannten Weise substituiert sein kann, mit Michael-Acceptoren der Formel (VI),

$BH=CH_2$ (VI)

in der

B für CN, CO-$R^{15}$ oder COOR$^{16}$ steht, wobei R15 für Methyl oder Ethyl und $R^{16}$ für Methyl, Ethyl, n- oder i-Propyl steht, umsetzt.

5.1,8-verbrückte 4-Chinolon-3-carbonsäuren und Derivate nach Anspruch 1 zur Verwendung in einem Verfahren zur therapeutischen Behandlung des menschlichen oder tierischen Körpers.

6. Arzneimittel, enthaltend 1,8-verbrückte 4-Chinolon-3-carbonsäuren und derivate nach Anspruch 1.

7. Verwendung von Verbindungen der Formel (I) gemäß Anspruch 1 zur Herstellung von Arzneimitteln.

8. Tierfutter bzw. Tierfutterzusätze, enthaltend Verbindungen der Formel (I) gemäß Anspruch 1.

## Claims

1. 1,8-Bridged 4-quinolone-3-carboxylic acids and derivatives of the formula (I)

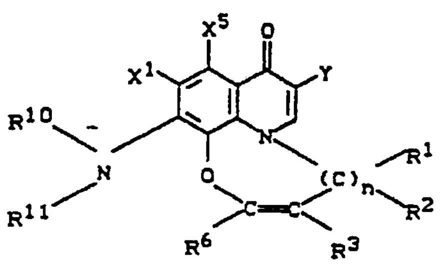

(I)

in which

Y represents a carboxyl group,

$X^1$ represents fluorine,

$X^5$ represents hydrogen and

$R^{10}$ and $R^{11}$, together with the nitrogen atom to which they are bonded, can form a 5- or 6-membered heterocyclic ring which can additionally contain an oxygen atom or the group N-$R^{12}$ as a ring member and which can optionally be mono- or disubstituted on the carbon atoms by $C_1$-$C_2$-alkyl, cyclohexyl, phenyl which is optionally substituted by chlorine, fluorine, methyl, phenyl, hydroxyl, methoxy, benzyloxy, nitro or piperidino, 2-thienyl or hydroxyl, wherein $R^{12}$ represents hydrogen, a branched or unbranched alkyl group with 1 to 3 carbon atoms, which can optionally be substituted by one or two hydroxyl groups, or a phenacyl radical, an oxalkyl radical with up to 4 carbon atoms or a radical COR$^{13}$, wherein $R^{13}$ denotes hydrogen or alkyl with one or two carbon atoms, $R_1$, $R_2$, $R_3$ and $R_6$ represent hydrogen or an alkyl group preferably having 1-6 carbon atoms, and $R_6$ can also represent phenyl and n denotes 0 or 1 and the compound ethyl 6,7-difluoro-9-oxo-9H-cyclohexa[1,2-b]-pyrido-[1′,2′,3′-de]1,4-benzoxazin-10-carboxylate and their pharmaceutically usable hydrates or salts, preferably alkali metal, alkaline earth metal, silver and anidinium salts and

their esters.

2. Process for the preparation of 1,8-bridged 4-quinolone-3-carboxylic acids and derivatives of the formula (I) according to Claim 1, characterized in that quinolonecarboxylic acid derivatives of the formula (II)

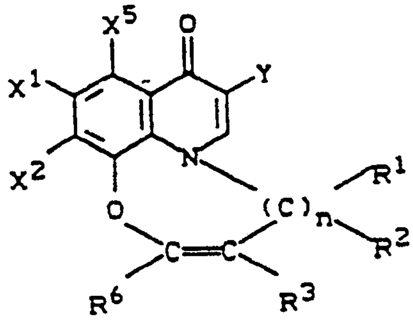

(II)

in which the radicals $X^1$, $X^5$, $R^1$, $R^2$, $R^3$ and $R^6$, Y and n have the abovementioned meanings and $X^2$ represents halogen, preferably chlorine or fluorine, are reacted with amines of the formula (III)

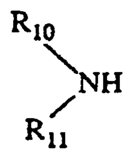

(III)

in which

$R^{10}$ and $R^{11}$ have the abovementioned meanings, if appropriate in the presence of acid-binding agents.

3. Process for the preparation of comptinds of the formula (I) according to Claim 1, characterized in that a 10-(1-piperazinyl) compound (where n=0) or 11-(1-piperazinyl compound where n=1 of the formula (IV)

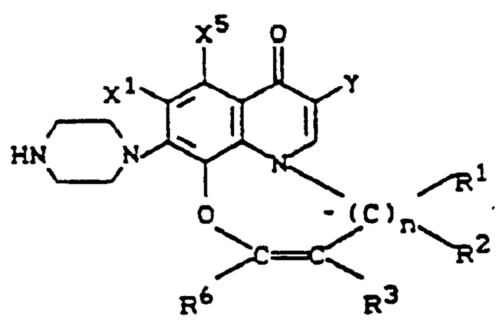

(IV)

in which

$X^1$, $X^5$, $R^1$, $R^2$, $R^3$ and $R^6$, Y and n have the abovementioned meanings, and the piperazinyl radical on the carbon atoms can be substituted in the manner described for $R^{10}$ and $R^{11}$, is reacted with compounds of the formula (V)

$R^{12}X$ (V)

in which

$R^{12}$ has the abovementioned meaning, but cannot be hydrogen, and

X denotes fluorine, chlorine, iodine, hydroxyl, acyloxy, ethoxy, phenoxy or 4-nitrophenoxy, if appropriate in the presence of acid-binding agents.

4. Process for the preparation of compounds of the formula (I) according to Claim 1, characterized in that 10-(1-piperazinyl)quinolonecarboxylic acid derivatives (n=0) or 11-(1-piperazinyl)quinolonecarboxylic acid derivatives (n=1) of the formula (IV) according to Claim 3, in which the piperazinyl radical can be substituted on the carbon atoms in the manner mentioned in Claim 3, are reacted with Michael acceptors of

the formula (VI)

$$B\text{-}CH{=}CH_2 \quad (VI)$$

in which

B represents CN, CO-$R^{15}$ or COO$R^{16}$, wherein $R^{15}$ represents methyl or ethyl and $R^{16}$ represents methyl, ethyl or n- or i-propyl.

5. 1,8-Bridged 4-quinolone-3-carboxylic acids and derivatives according to Claim 1 for use in a method of therapeutic treatment of the human or animal body.

6. Medicaments containing 1,8-bridged 4-quinolone-3-carboxylic acids and derivatives according to Claim 1.

7. Use of compounds of the formula (I) according to Claim 1 for the preparation of medicaments.

8. Animal feeds and animal feed additives containing compounds of the formula (I) according to Claim 1.

**Revendications**

1. Acides 4-quinolone-3-carboxyliques pontés sur les positions 1,8 et leurs dérivés de formule (I)

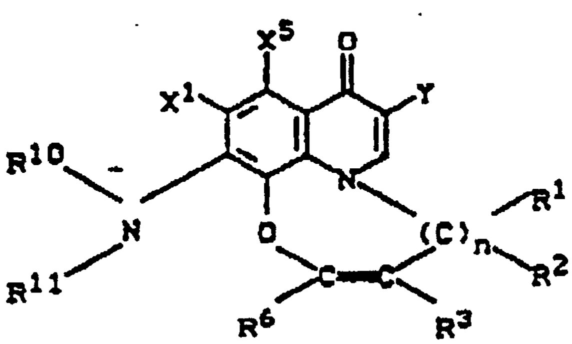

(I)

dans laquelle

Y représente un groupe carboxyle,

$X^1$ représente un atome de fluor,

$X^5$ représente l'hydrogène,

$R^{10}$ et $R^{11}$ peuvent former ensemble avec l'atome d'azote auquel ils sont liés, un noyau hétérocyclique à 5 ou 6 chaînons qui, comme terme cyclique, peut contenir de plus un atome d'oxygène ou un groupe N-$R^{12}$ et peut éventuellement porter sur ses atomes de carbone un ou deux substituant(s) consistant en un groupe alkyle en $C_1$-$C_2$, un groupe cyclohexyle, un groupe hydroxy, un groupe 2-thiényle ou un groupe phényle éventuellement substitué par le chlore, le fluor, un groupe méthyle, phényle, hydroxy, méthoxy, benzyloxy, nitro ou pipéridino, $R^{12}$ représentant l'hydrogène, un groupe alkyle ramifié ou non ramifié avec 1 à 3 atomes de carbone pouvant éventuellement porter comme substituant(s) un ou deux groupes hydroxy, un reste phénacyle, un reste oxalkyle possédant jusqu'à 4 atomes de carbone ainsi qu'un reste COR$^{13}$, $R^{13}$ représentant l'hydrogène ou un groupe alkyle avec un ou deux atomes de carbone,

$R_1$, $R_2$, $R_3$ et $R_6$ peuvent représenter l'hydrogène, un groupe alkyle comportant de préférence 1 à 6 atomes de carbone, $R_6$ pouvant également représenter un groupe phényle, et n signifie 0 ou 1, ainsi que le composé ester éthylique d'acide 6,7-difluor-9-oxo-9H-cyclohexa-[1,2-b]-pyrido-[1',2',3'-de]1,4-benzoxazine-10-carboxylique

et leurs hydrates ou leurs sels utilisables dans le domaine pharmaceutique, de préférence les sels de métaux alcalins, de métaux alcalinoterreux, d'argent et de guanidium, ainsi que leurs esters.

2. Procédé pour la fabrication d'acides 4-quinolone-3-carboxyliques pontés sur les positions 1,8 et leurs dérivés de formule (I) selon la revendication 1, caractérisé en ce que l'on fait réagir des dérivés d'acide quinolone-carboxylique de formule (II)

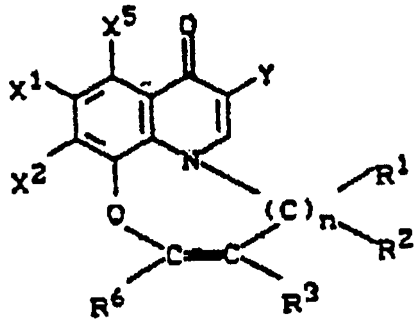

(II)

dans laquelle les restes $X^1$, $X^5$, $R^1$, $R^2$, $R^3$ et $R^6$, Y ainsi que n ont les significations décrites ci-dessus, et $X^2$ représente un halogène, de préférence le chlore ou le fluor, avec des amines de formule (III)

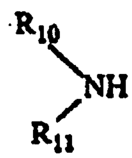

(III)

dans laquelle

$R^{10}$ et $R^{11}$ ont les significations décrites ci-dessus, éventuellement en présence de fixateurs d'acide.

3. Procédé pour la fabrication de composés de formule (I) selon la revendication 1, caractérisé en ce qu'on fait réagir un composé de 10-(1-pipérazinyl) (n = 0) ou un composé 11-(1-pipérazinyl) (n=1) de formule (IV)

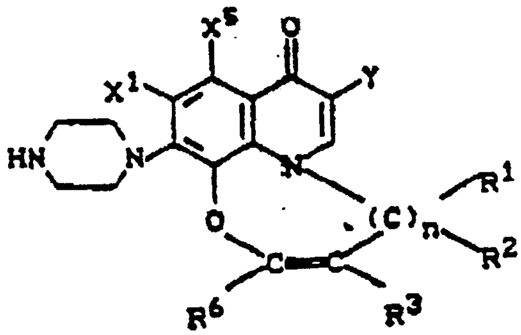

(IV)

dans laquelle

$X^1$, $X^5$, $R^1$, $R^2$, $R^3$ et $R^5$, Y et n ont les significations décrites ci-dessus, et le reste pipérazinyle sur les atomes de carbone peut être substitué de la manière décrite pour $R^{10}$ et $R^{11}$,
avec des composés de formule (V)

$$R^{12}X \quad (V)$$

dans laquelle

$R^{12}$ possède la signification décrite ci-dessus mais ne peut pas être l'hydrogène, et

X représente le fluor, le chlore, l'iode, un groupe hydroxy, acyloxy, éthoxy, phénoxy, 4-nitrophénoxy, éventuellement en présence de fixateurs d'acide.

4.Procédé pour la fabrication de composés de formule (I) selon la revendication 1, caractérisé en ce qu'on fait réagir des dérivés d'acide 10-(1-pipérazinyl)quinolonecarboxylique (n = 0) ou des dérivés d'acide 11-(1-pipérazinyl)-quinolonecarboxylique (n = 1) de formule (IV) selon la revendication 3, dans laquelle le

reste pipérazinyle sur les atomes de carbone peut être substitué de la manière décrite dans la revendication 3, avec des accepteurs de Michael de formule (VI),

$$B\text{–}CH{=}CH_2 \quad (VI)$$

dans laquelle

B représente un groupe CN, CO-$R^{15}$ ou COOR$^{16}$, $R^{15}$ représentant un groupe méthyle ou éthyle et $R^{16}$ représentant un groupe méthyle, éthyle, n- ou i-propile.

5. Acides 4-quinolone-3-carboxyliques pontés sur les positions 1,8 et leurs dérivés selon la revendication 1 pour l'application dans un procédé pour le traitement thérapeutique du corps humain ou animal.

6. Médicament contenant des acides 4-quinolone-3-carboxyliques pontés sur les positions 1,8 et dérivés selon la revendication 1.

7. Utilisation des composés de formule (I) selon la revendication 1 pour la fabrication de médicaments.

8. Aliment pour animaux ou additifs pour aliments pour animaux contenant les composés de formule (I) selon la revendication 1.